# EUROPÄISCHE PATENTANMELDUNG

(11) **EP 1 350 541 A1**
(43) Veröffentlichungstag der Anmeldung: **08.10.2003**
(21) Anmeldenummer: 02007648.5
(22) Anmeldetag: 04.04.2002
(51) Int. Cl.: A61P 15/12, A61K 31/565

(54) **Verwendung von einem Dehydroepiandrosteron und einem Estradiol Derivat zur Herstellung einer pharmazeutischen Zubereitung zur Behandlung klimakterischer Beschwerden in der Postmenopause**

(71) Anmelder: Jenapharm GmbH & Co. KG, 07745 Jena (DE)
(72) Erfinder: Oettel, Michael, Prof, Dr., 07743 Jena (DE); Patchev, Vladimir, Prof, Dr., 07749 Jena (DE); Hübler, Doris, Dr., 07407 Schmieden (DE); Fricke, Sabine, Dr., 07749 Jena (DE)
(74) Vertreter: Störle, Christian, Dr.

(57) **Zusammenfassung**

Die Anmeldung bezieht sich auf Arzneimittel zur Behandlung der klimakterischen Beschwerden in der Postmenopause, das umfaßt
a) Steroid der Formel (1) 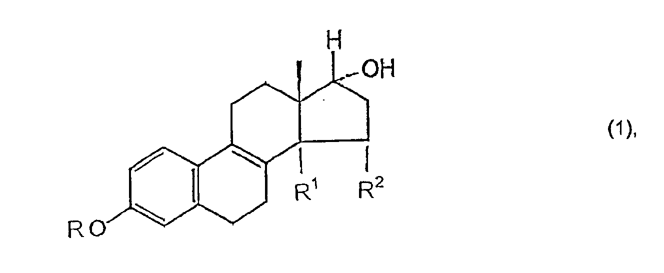 in der
   R für Wasserstoff oder ein C₁-C₄-Alkyl und
   R¹ und R² für Wasserstoff stehen oder R¹ und R² identisch sind und CH₂ bedeuten, sowie
b) Dehydroepiandrosteron und/oder einen Dehydroepiandrosteron-Ester und/oder ein anderes Dehydroepiandrosteron-Derivat.

## Beschreibung

Die Erfindung bezieht sich auf ein Arzneimittel zur Behandlung der klimakterischen Beschwerden in der Postmenopause.

Beim Klimakterium, den Wechseljahren der Frau, handelt es sich um die Übergangsphase von der vollen Geschlechtsreife zum Senium der Frau. Das Erlöschen der zyklischen Ovarialfunktion, die letzte Regel (Menopause), fällt in diese Zeit hinein. Der Abschnitt des Klimakteriums nach der Menopause wird als Postmenopause bezeichnet, der davor als Prämenopause. Etwa ein bis zwei Drittel der Frauen im Klimakterium leiden unter Beschwerden, die behandlungsbedürftig sind. Die klimakterischen Beschwerden in der Postmenopause sind vor allem vegetative und psychische Störungen (Ausfallerscheinungen), Hitzewallungen, Kälteschauer, Schweißausbrüche, Schwindel, Herzklopfen und pektanginöse Beschwerden, Schlaflosigkeit, Angstgefühle, Abnahme der Leistungsfähigkeit, Vergeßlichkeit, Antriebsschwäche, Stimmungslabilität, Depressionen und Reizbarkeit (Pschyrembel Klinisches Wörterbuch, Berlin, New York, 1986, Seite 861).

Zur Behandlung dieser Beschwerden wird die Substitution mit Oestrogenen vorgeschlagen (Grady D, Rubin SM, Petitti DB et al., Hormone replacement therapy to prevent disease and prolong life in postmenopausal women, Ann Intern Med 1992; 1117: 1016-1037).

Um jedoch bei nicht-hysterektomierten Frauen den proliferativen Effekt einer allgemeinen Estrogensubstitution auf das Endometrium (Gefahr der Hyperplasie und des Endometrium-Karzinoms) zu unterbinden, werden Gestagene zugesetzt (Pike MC, Ross RK, Progestogens and Menopause: Epidemiological Studies of risks of endometrial and breast cancer, Steroids 2000; 65: 659-664). Gegenwärtig wird jedoch diskutiert, ob der Gestagenzusatz nicht das Risiko für das Auftreten eines Mamma-Karzinoms erhöht (Persson, Estrogens in the causation of breast, endometrial and ovarian cancers - evidence and hypotheses from epidemiological findings: J. Steroid Biochem Mol Biol 2000; 74:357-364; Santen RJ, Pinkerton J, McCartney C, Petroni GR, Risk of breast cancer with progestins in combination with estrogen as hormone replacement therapy, J. Clin Endocrinol Metale 2001; 86: 16-23).

Darüber hinaus gibt es eine Gruppe postmenopausaler Frauen die zusätzlich zu Oestrogenen und Gestagenen die Zufuhr von Androgenen benötigen, da bei ihnen trotz Oestrogen-Gestagen-Substitution die Symptome eines Androgenmangels (Mangel an männlichen Geschlechtshormonen) zu beobachten sind, wie verminderte Libido, Haarausfall, Kaurosis vulvae, verminderte Knochendichte, dünne und trockene Haut (Knochenhauer E, Azziz R, Ovarian hormones and adrenal androgens during a woman's life span, J Am Acad Dermatol 2001; 45: 5105-15).

Die Akzeptanz ein Langzeit-Androgensubstitution hängt im Wesentlichen davon ab, ob ein entsprechendes oral oder transdermal verabreichbares Hormonpräparat zur Verfügung steht.

Das Androgen Testosteron kann per se nicht oral verabreicht werden, da durch einen ausgeprägten Leber-Firstpass-Effekt keine systemische Wirkung erreicht wird. Gegenwärtig sind nur zwei Androgenpräparate zur oralen Gabe bekannt, nämlich 17α-Methyltestosteron und Testosteronundecanoat. Methyltestosteron besitzt eine deutliche Leber-Toxizität und wurde deshalb in verschiedenen europäischen Ländern vom Markt genommen. Testosteronundecanoat muß hoch dosiert und mehrmals täglich verabreicht werden. Der Hauptnachteil dieses Androgens ist jedoch eine hohe inter- und intraindividuelle Schwankungsbreite in der Resorption, die eine genaue und gleichbleibende Dosierung nahezu unmöglich macht.

In den USA ist eine Estrogen/Androgen-Kombination (Estratest®) auf dem Markt, bei der 17β-Estradiol mit 17α-Methyltestosteron zusammen oral verabreicht wird. Diese Kombination hat u.a. den Nachteil, daß die Gebärmutterschleimhaut ungeschützt gegenüber den proliferativen Aktivitäten des Estrogens bleibt und darüber hinaus toxikologisch bedenkliches Androgen eingesetzt wird.

Der vorliegenden Erfindung liegt somit die Aufgabe zugrunde, ein Arzneimittel bereitzustellen, mit dem die vorgenannten Nachteile vermieden werden können.

Erfindungsgemäß wird dies erreicht durch ein Arzneimittel zur Behandlung der klimakterischen Beschwerden in der Postmenopause, das umfaßt
a) Steroid der Formel (1) 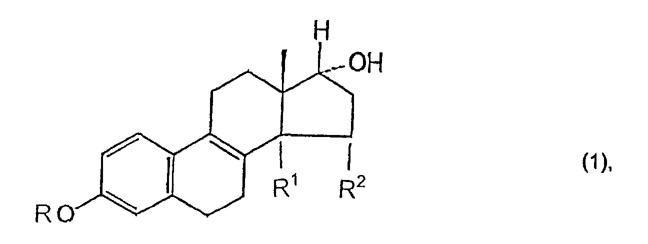 in der
   R für Wasserstoff oder ein C₁-C₄-Alkyl und
   R¹ und R² für Wasserstoff stehen oder R¹ und R² identisch sind und CH₂ bedeuten, sowie
b) Dehydroepiandrosteron (DHEA) und/oder Dehydroepiandrosteron-Ester und/oder andere Derivate des DHEA.

Die Aufgabe wird demgemäß gelöst durch Verwendung eines dissoziierten Estrogens, das am Uterus keine proliferative Aktivität, in anderen Organen, z.B. im zentralen Nervensystem, aber die gleiche Wirkung wie das natürliche Estrogen 17β-Estradiol zeigt. Dadurch ist die zusätzliche Gabe eines Gestagens mit dem erhöhten Risiko für ein Mamma-Karzinom nicht notwendig.

Durch Verzicht auf die Gestagen-Komponente wird gleichzeitig ausgeschlossen, daß durch die bekannten antiestrogenen und antiandrogenen Eigenschaften der Gestagene der Estrogen- bzw. Androgen-Benefit bei postmenopausalen Frauen reduziert wird. Weiterhin ergeben sich technologische Vorteile, da derartige Mehrkomponenten-Arzneimittel galenisch schwierig zu handhaben sind.

Femer wird die Aufgabe gelöst durch Verwendung eines oral wirksamen Prodrugs für Testosteron, das sich durch gute Verträglichkeit und sichere androgene Wirksamkeit speziell bei Frauen auszeichnet. Es wurde gefunden, daß diese Forderungen an ein derartiges Prodrug von Dehydroepiandrosteron (DHEA) erfüllt wird. Durch eine Verstoffwechselung des DHEA zu Testosteron "nach Bedarf" (on demand) sind Überdosierungen praktisch nicht möglich. Selbst bei hohen Dosierungen von 100 mg/Tag oral konnten keine unerwünschten Virilisierungserscheinungen beobachtet werden. (Arit W, Callies F, van Vlijmen JC, Koehler I, Reincke M, Bidlingmaier M, Hübler D, Oettel M, Ernst M, Schulte HM, Allolio B: Dehydroepiandrosteron replacement in women with adrenal insufficiency. New Engl J Med 1999; 341: 1013-1020).

Die vorliegende Erfindung wird nun unter Bezugnahme auf die Abbildungen erläutert.

Das klinische Bild eines Estrogenmangels in der Postmenopause wird hauptsächlich durch Ausfallserscheinungen im Zentralen Nervensystem (ZNS) geprägt (z.B. Hitzewallungen, depressive Zustände, Antriebsarmut, Nachlassen cognitiver Leistungen, die später z.B. zu einem Morbus Alzheimer führen können). Aus diesem Grunde ist der pharmakologische Nachweis von Estrogenwirkungen im ZNS und der pharmakologische Beleg für fehlende estrogene Wirkungen am Genitalapparat für die estrogene Komponente im Sinne des vorliegenden Patentanspruchs von besonderer Bedeutung.

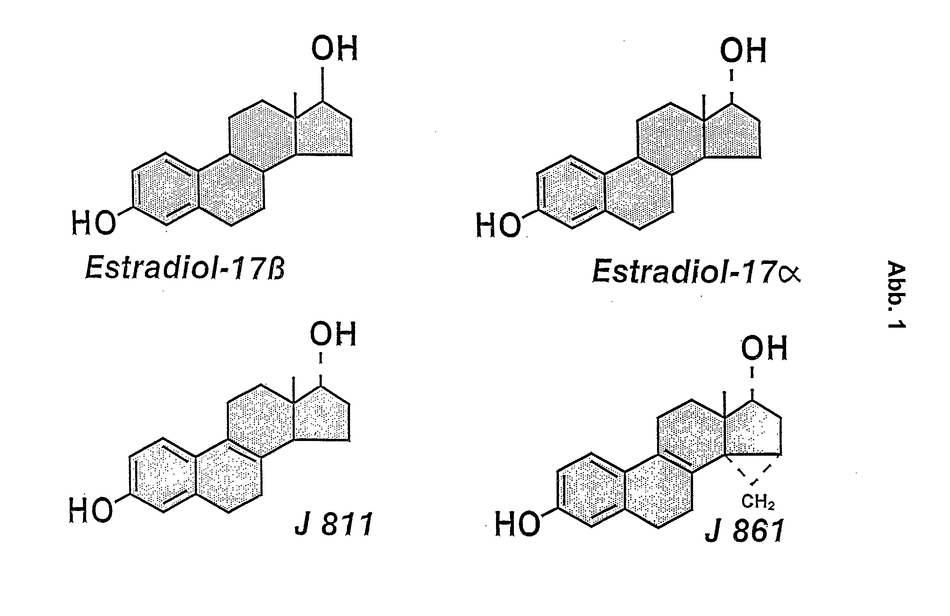

In den Abbildungen 1 bis 4 wird nun diese überraschende Dissoziation an Beispielen erläutert.

Abbildung 1 zeigt die chemische Struktur der beiden Beispielverbindungen J 811 und J 861 und die der Referenz-Estrogene 17β-Estradiol und 17α-Estradiol.

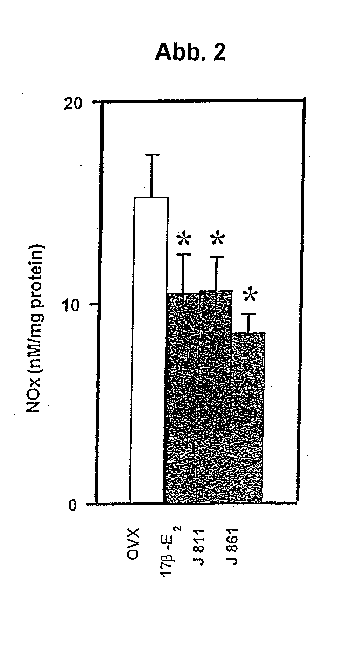

Eine der typischen Estrogenwirkungen im Gehirn ist die Hemmung der induzierbaren Stickstoffmonoxid-Oxidase (iNOS). Aus Abbildung 2 wird ersichtlich, daß bei weiblichen, ovarektomierten Ratten die Hemmung der Freisetzung von NO durch die beiden Beispielverbindungen J 811 und J 861 genauso stark wie bei dem wichtigsten körpereigenen Estrogen, dem 17β-Estradiol (17β-E₂), ist. Die Kontrollgruppe sind ovarektomierte Tiere (OVX), die nur mit dem Lösungsmittel behandelt worden sind. (* Bedeutet statistisch signifikant gegenüber der Kontrollgruppe (p<0.01))

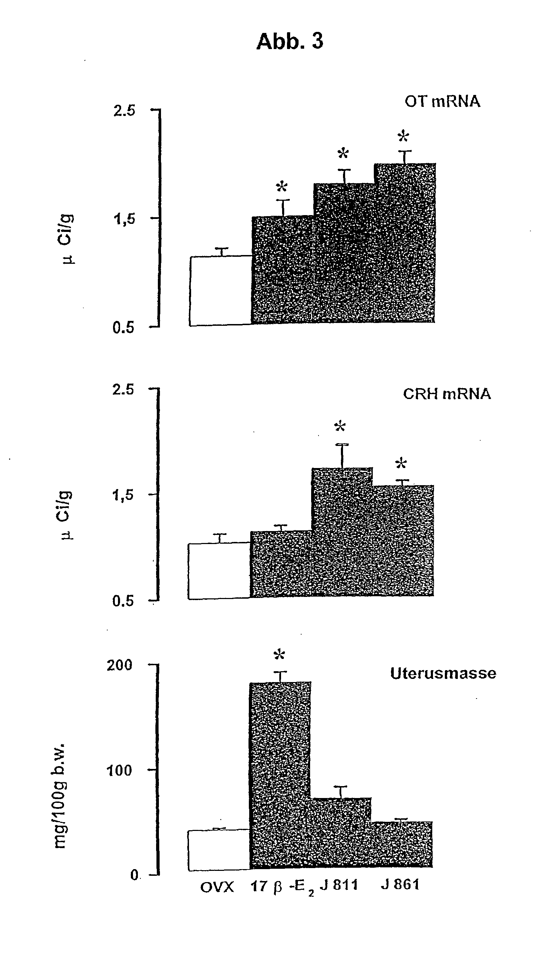

In Abbildung 3 werden zwei weitere Beispiele für typische Estrogenwirkungen im Gehirn von weiblichen, ovarektomierten Ratten dargestellt. Die Induktion der estrogenabhängigen Gene für Oxytocin (Nachweis der OT mRNA) und für Corticotropin-Releaseinghormon (Nachweis der CRH mRNA) gelingt sowohl mit dem natürlichen Estrogen 17β-Estradiol (17β-E₂) als auch mit den beispielhaften Verbindungen J 811 und J 861. Aber im Gegensatz zu 17β-Estradiol ist eine estrogene Wirkung auf den Uterus (hier: Zunahme der Uterusmasse) nicht nachweisbar. (* Bedeutet statistisch signifikant gegenüber der Kontrollgruppe (p<0.01))

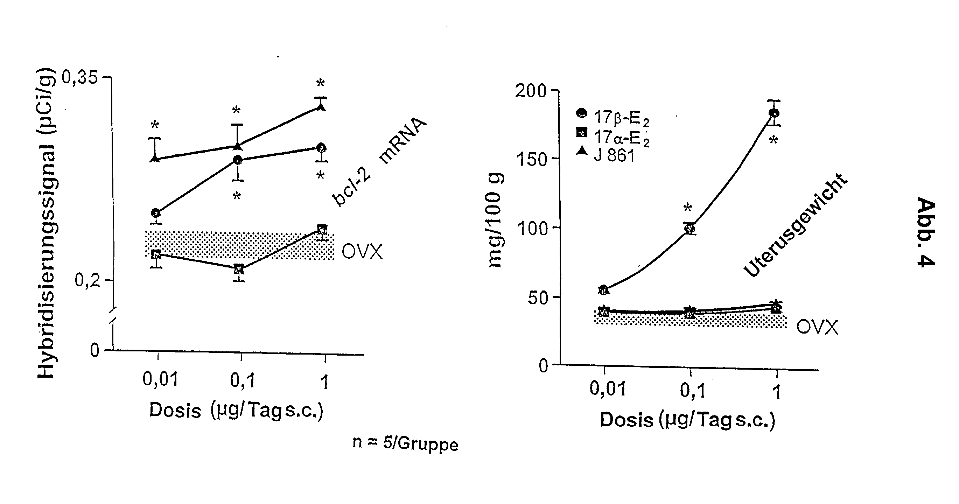

In Abbildung 4 ist dargestellt, daß sowohl die Applikation von 17β-Estradiol (17β-E₂) als auch die der beispielhaften Verbindung J 861 die Expression des antiapoptotischen Gens bcl-2 im Hippocampus von weiblichen, ovarektomierten Ratten signifikant erhöht (im Gegensatz zur Kontrolle (OVX) und zu Tieren, die mit dem sehr schwachen Estrogen 17α-Estradiol behandelt worden sind). (* Bedeutet statistisch signifikant gegenüber der Kontrollgruppe (p<0.01)) Im Gegensatz zu 17β-Estradiol besitzt aber J 861 keinerlei Wirkung auf den Uterus.

Bezüglich der androgenen Komponente b) der erfindungsgemäßen Arzneimittels sei darauf hingewiesen, daß für den Nachweis der Umwandlung von DHEA zu Testosteron Tierexperimente nur eine begrenzte Aussagekraft haben, da bei den meisten Tierarten DHEA als körpereigenes Steroid nicht nachweisbar ist.

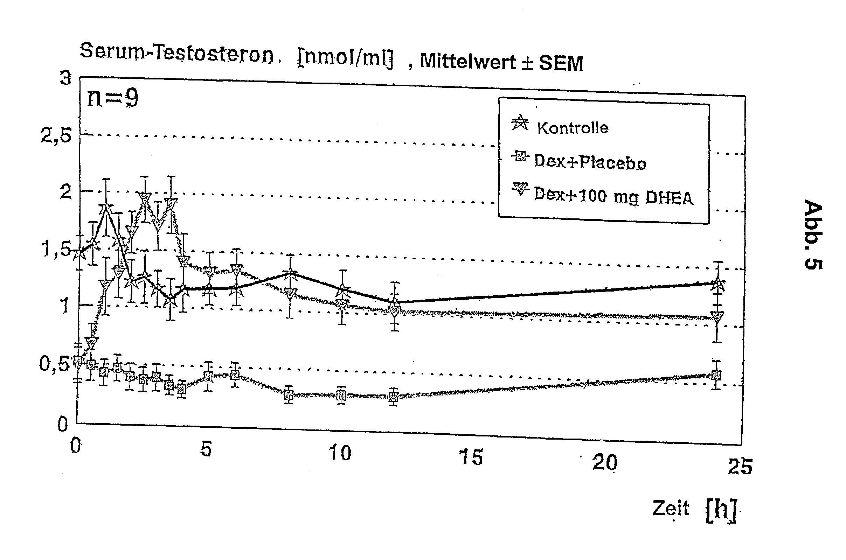

In Abbildung 5 werden daher Ergebnisse einer klinischen Studie mit gesunden Frauen dargestellt. Zur kurzzeitigen Unterdrückung der körpereigenen Steroidproduktion erhielten die Probandinnen einmalig Dexamethason. Der Abbildung kann entnommen werden, daß die orale Einnahme von 100 mg DHEA die Serum-Testosteronkonzentrationen auf das Niveau der unbehandelten Kontrollen hebt. Damit ist der Nachweis geführt, daß DHEA bei Frauen nach oraler Aufnahme im Körper zu dem sehr wirksamen Androgen Testosteron umgewandelt wird, d.h. DHEA ist bei Frauen ein Prodrug für Testosteron.

Wie vorstehend ausgeführt wurde, kann das Steroid a) einen C₁-C₄-Alkylrest aufweisen. Der C₁-C₄-Alkylrest bedeutet dabei insbesondere Methyl, Ethyl und Butyl.

Die OH-Gruppe am C17 des Steroid-Grundgerüsts ist insbesondere α-ständig. Die MethylGruppe am C13 ist insbesondere β-ständig.

Wie bereits weiter oben erläutert wurde, können die Substituenten R¹ und R² identisch sein und dabei für die CH₂-Gruppe stehen. Dies bedeutet, daß die Kohlenstoff-Atome an Positionen 14 und 15 des Steroid-Grundgerüsts mit dieser CH₂-Gruppe einen Cyclopropanring bilden. Die Substituenten R¹ und R² sind insbesondere α-ständig.

Durch vorgenannte Konfigurationen werden besonders gute Wirkungen des erfindungsgemäßen Arzneimittels erzielt.

Bei den vorgenannten Steroiden handelt es sich um an sich bekannte Verbindungen, die bisher aber für andere Zwecke eingesetzt wurden. So beschreibt die DE 42 39 945 A1 "14α, 15α-Methylensteroide und Verfahren zu ihrer Herstellung", wie sie auch vorstehend angegeben sind, wobei in dieser deutschen Offenlegungsschrift nur die fertilitätshemmende Wirkung dieser Steroide offenbart ist.

Die erfindungsgemäßen Arzneimittel weisen zusätzlich zu den Steroiden a) als Komponente b) Dehydroepiandrosteron (DHEA) und/oder Dehydroepiandrosteron-Ester und/oder andere DHEA-Derivate auf. DHEA hat 1 Hydroxyl-Gruppe, die mit Säuren verestert werden kann.

Vertreter dieser Säuren sind anorganische Säuren, wie Schwefelsäure, Phosphorsäure, Salpetersäure, Halogenwasserstoffsäure, insbesondere HCI, oder organische Säuren, wie Fettsäuren, insbesondere ungesättigte Fettsäuren mit 1 bis 6 Kohlenstoffatomen. Als DHEA-Ester hat sich DHEA-Sulfat (DHEAS) als besonders günstig erwiesen, da es natürlich vorkommt und eine besonders gute Wirkung entfaltet.

Es wurde nun überraschenderweise gefunden, daß das pharmakologische und pharmakokinetische Profil des erfindungsgemäßen Arzneimittels geeignet ist, klimakterischen Beschwerden in der Postmenopause infolge gleichzeitigen Mangels an Estrogenen und Androgenen, wie z.B. vegetative und psychische Störungen (Ausfallerscheinungen), Hitzewallungen, Kälteschauer, Schweißausbrüche, Schwindel, Herzklopfen und pektanginöse Beschwerden, Schlaflosigkeit, Angstgefühle, Abnahme der Leistungsfähigkeit, Vergeßlichkeit, Antriebsschwäche, Stimmungslabilität, Depressionen und Reizbarkeit zu behandeln, und zwar ohne daß die vorstehend geschilderten Nachteile aus dem Stand der Technik in Kauf genommen werden müssen.

Als besonders vorteilhaft für die erfindungsgemäßen Arzneimittel hat sich dabei herausgestellt, daß die Steroide der Formel (1) keine oder nur eine schwache Wirkung am Uterus zeigen. Durch dieses ganz spezielle und besonders vorteilhafte Wirkprofil der erfindungsgemäßen Verbindungen besteht keine Gefahr für Hyperplasien des Endometriums und damit kein erhöhtes Risiko, an einem Endometriumkarzinom zu erkranken.

Die im erfindungsgemäßen Arzneimittel als Komponenten b) eingesetzten DHEA und/oder DHEA-Ester, sind besonders gut verträglich, d.h. unerwünschte Nebenwirkungen, wie z.B. Leber-Toxizität, werden nicht beobachtet. Des weiteren können sie oral und transdermal besonders gut verabreicht werden, was von Vorteil ist, da diese Applikationsart nicht unter Aufsicht von medizinisch geschultem Personal erfolgen muß, wie beispielsweise bei Injektionen oder Infusionen. Die körpereigene Testosteron-Blutserumkonzentration wird durch DHEA und DHEA-Ester, insbesondere DHEAS, in ausreichendem Maß angehoben, um die gewünschten therapeutischen Effekte zur Behandlung der klimakterischen Beschwerden in der Postmenopause zu erreichen. Des weiteren ist bei diesen Verbindungen vorteilhafterweise die individuelle Schwankungsbreite in der Resorption nicht sehr groß.

Das erfindungsgemäße Arzneimittel weist weiterhin den Vorteil auf, daß Gestagene, wie sie üblicherweise bei der Hormonsubstitutionsbehandlung der klimakterischen Beschwerden postmenopausaler Frauen eingesetzt werden, nicht erforderlich sind. Dies bedeutet, daß die unerwünschten Nebenwirkungen, die durch Gestagene verursacht werden, wie ein erhöhtes Mamma-Karzinom-Risiko und die Förderung von Venenthrombosen, nicht beobachtet werden.

In einer bevorzugten Ausführungsform des erfindungsgemäßen Arzneimittels weist das Steroid (a) die in der Formel (2) angegebene Struktur 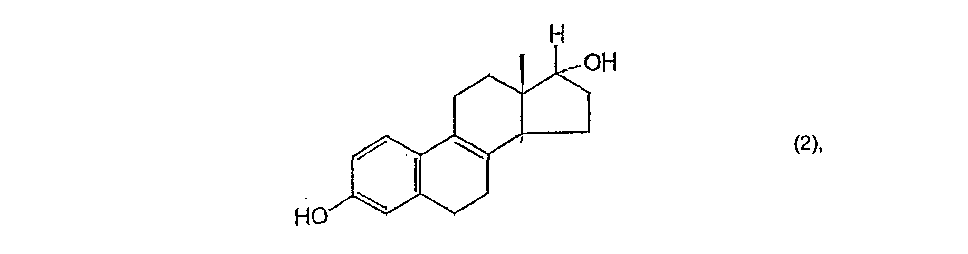 (Estra-1,3,5(10),8-tetraen-3,17α-diol), oder die der Formel (3) 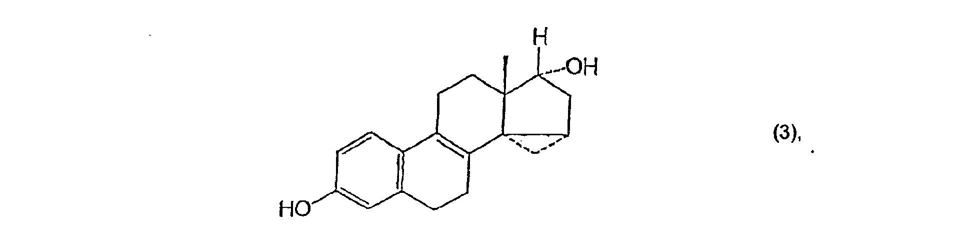 (14α, 15α-Methylenestra-1,3,5(10),8-tetraen-3,17α-diol) auf.

Die erfindungsgemäßen Arzneimittel sind zur oralen und parenteralen, einschließlich topischen, rektalen, subcutanen, intravenösen, intramuskulären, intraperitonealen, intranasalen, intravaginalen, intrabukkalen oder sublingualen Applikation geeignet. Neben üblichen Trägerund Verdünnungsmitteln enthalten sie als Wirkstoff ein vorgenanntes Steroid und DHEA und/oder einen DHEA-Ester und/oder andere Derivate des DHEA.

Die erfindungsgemäßen Arzneimittel werden mit den üblichen festen oder flüssigen Trägerstoffen oder Verdünnungsmitteln und den üblicherweise verwendeten pharmazeutischtechnischen Hilfsstoffen entsprechend der gewünschten Applikationsart mit einer geeigneten Dosierung des Wirkstoffs in bekannter Weise hergestellt.

Die Dosierung beträgt für DHEA, den DHEA-Ester und das andere DHEA-Derivat insbesondere 10 mg bis 200 mg pro Arzneimittelformulierung, sowie für das neurotrope Steroid insbesondere 0,05 mg bis 10 mg pro Arzneimittelformulierung, vor allem etwa 0,2 mg.

Vorzugsweise liegt das erfindungsgemäße Arzneimittel in einer solchen Form vor, daß es dem Patienten oral oder transdermal verabreicht werden kann. Diese Applikationsformen haben den Vorteil, daß sie leicht handhabbar sind und vom Patienten angewendet werden können, ohne daß die Aufsicht von medizinisch geschultem Personal notwendig ist.

Als oral verabreichbare Formulierung können übliche Tabletten Steckkapseln oder Weichgelantinekapseln, insbesondere mit mikronisiertem Wirkstoff, eingesetzt werden.

Als oral verabreichbare Formulierung kommen insbesondere Weichgelatinekapseln zur Anwendung, da damit die Wirkstoffe besonders gut verabreicht werden können. Je nach Dosierung des Wirkstoffs kann die Kapselgröße zwischen 5 minims/oval oder oblong und 14 minims/oblong bzw. 10 minims/oval liegen.

Für die Herstellung des wirkstoffhaltigen Kapselfüllguts der Weichgelatinekapseln kommen zwei grundsätzlich unterschiedliche Varianten in Betracht, nämlich die mit hydrophilem und die mit hydrophobem Füllgut. Bei der Variante mit hydrophilem Füllgut wird das Steroid a) zusammen mit einem Stabilisator, insbesondere Ascorbinsäure, in einer Mischung aus Lösungsmitteln und/oder Lösungsvermittlern, z.B. Partialglyceriden, Propylenglycol und Macrogolglycerol-hydroxystearat gelöst. Danach wird DHEA und/oder dessen Ester zugesetzt. Je nach Dosierung des DHEA entsteht eine Lösung oder eine Suspension. Im Falle einer Suspension kann zur Viskositätserhöhung ein üblicher (hydrophiler) Matrixbildner, beispielsweise makromolekulare Schleimstoffe wie Hydroxyethylcellulose, Natriumcarboxymethylcellulose oder PEG, in den pharmazeutisch üblichen Konzentrationen zugesetzt werden. Bei hydrophobem Füllgut wird als Grundlage dafür ein mittelkettiges Triglycerid verwendet, das in eine ethanolische Lösung des Steroids a) und einem Stabilisator, beispielsweise Ascorbinsäure, eingetragen wird. Zu dieser Mischung wird dann DHEA und/oder ein DHEA-Ester unter Rühren fein eingetragen. Es entsteht eine Suspension, die durch pharmazeutisch übliche hydrophobe Matrixbildner, beispielsweise Aerolsil oder Aluminiumstearat, noch verstärkt werden kann. Die so hergestellten Lösungen bzw. Suspensionen können nach dem bekannten Scherer-Verfahren zu Weichgelatinekapseln verarbeitet werden. Dabei kann sowohl die übliche Standardgelatine als auch succinylierte Gelatine zum Einsatz kommen.

Gemäß den vorsehenden Ausführungen liegen die Wirkstoffe Steroid und DHEA und/oder DHEA-Ester in einer Applikationsform, z. B. Tablette, Weichgelatinekapsel oder transdermales therapeutisches System zusammen vor, d. h. die Applikationsform enthält beide Wirkstoffgruppen.

Als Arzneimittel im Sinne der vorliegenden Erfindung werden auch Präparate mit zwei Applikationsformen verstanden, wobei eine Applikationsform das Steroid und die andere Applikationsform DHEA und/oder den Ester und/oder ein anderes Derivat davon enthält. Die Applikationsformen können gleich oder unterschiedlich sein. Es handelt sich hierbei also um ein Kombinationspräparat, in dem die Arzneimittel zwar in einem räumlichen Nebeneinander vorliegen, jedoch die Wirkstoffe (Steroid einerseits bzw. DHEA und/oder dessen Ester andererseits) in Applikationsformen körperlich voneinander getrennt sind. Dadurch kann in günstiger Weise ausgeschlossen werden, daß eine negative chemische Wechselwirkung der Arzneimittelwirkstoffe eintritt. Des weiteren kann die Verabreichung der Wirkstoffe zu unterschiedlichen Zeitpunkten erfolgen, beispielsweise kann ein Wirkstoff abends und der andere Wirkstoff morgens gegeben werden, wodurch negative physiologische Wechselwirkungen der Wirkstoffe vermieden werden können.

Ein solches Kombinationspräparat soll anhand einer oral verabreichbaren Applikationsform, beispielsweise einer Tablette oder einer Weichgelatinekapsel, und einem transdermalen therapeutischen System beispielhaft veranschaulicht werden. Liegt das Steroid in oral verabreichbarer Form, wie einer Tablette oder einer Weichgelatinekapsel vor, kann DHEA oder dessen Ester ebenfalls in einer oral verabreichbaren Form oder als transdermales therapeutisches System vorliegen. Das Kombinationspräparat enthält in einem solchen Fall beispielsweise zwei Tabletten bzw. eine Tablette und ein transdermales Pflaster. Liegt auf der anderen Seite das Steroid als transdermales therapeutisches System vor, kann DHEA bzw. dessen Ester ebenfalls als transdermales Pflaster oder in Form einer Tablette oder Weichgelatinekapsel vorliegen.

Die zwei Applikationsformen können in 1 Arzneimittelpackung enthalten sein.

Eine hinreichende Haltbarkeit der an sich sehr instabilen Steroide in Arzneimittelformulierungen wird insbesondere erreicht, wenn ein Stabilisator vorliegt. Als besonders geeigneter Stabilisator hat sich dabei überraschenderweise Ascorbinsäure erwiesen, da es besonders wirksam ist. In einer bevorzugten Ausführungsform des erfindungsgemäßen Arzneimittels wird deshalb das Steroid mit Ascorbinsäure stabilisiert. Andere Stabilisatoren, wie Butylhydroxyanisol, Butylhydroxytoluol oder Ascorbylpalmitat, waren hingegen in ihrer stabilisierenden Wirkung deutlich weniger effektiv. Die Konzentration des Stabilisators Ascorbinsäure ist vom Gehalt des Steroids abhängig. Je nach dessen Gehalt werden bei Weichgelatinekapseln 0,1 % bis 1 % Ascorbinsäure, bezogen auf die Füllmasse, eingesetzt.

Die folgenden Beispiele erläutern die Erfindung, ohne sie darauf einzuschränken.

### Beispiel 1: Herstellung von Weichgelatinekapseln enthaltend DHEA und 14α, 15α-Methylenestra-1,3,5(10),8-tetraen-3,17α-diol

Die Verbindung 14α,15α-Methylenestra-1,2,5(10),8-tetraen-3,17α-diol wird mit "Methylendiol-Steroid" bezeichnet.

### 1. hydrophiles Füllgut

| **Bestandteile** | **Zusammensetzung/Kapsel** |
|---|---|
| Methylendiol-Steroid | 0,2 mg |
| Dehydroepiandrosteron (DHEA) | 50,0 mg |
| Ascorbinsäure | 2,5 mg |
| Propylenglycol | 25,0 mg |
| Macrogolglycerol-hydroxystearat (Cremophor® RH 40) | 73,0 mg |
| Mittelkettige Partialglyceride (Imwitor® 742) | 149,3 mg |
| Gesamtmasse 300,0 mg | |

### 2. hydrophiles Füllgut mit Suspensionsverstärker

| **Bestandteile** | **Zusammensetzung/Kapsel** |
|---|---|
| Methylendiol-Steroid | 0,2 mg |
| Dehydroepiandrosteron (DHEA) | 50,0 mg |
| Ascorbinsäure | 2,5 mg |
| Propylenglycol | 25,0 mg |
| Macrogolglycerol-hydroxystearat (Cremophor® RH 40) | 73,0 mg |
| Hydroxyethylcellulose | 7,5 mg |
| Mittelkettige Partialglyceride (Imwitor® 742) | 141,8 mg |
| Gesamtmasse 300,0 mg | |

### 3. hydrophobes Füllgut

| **Bestandteile** | **Zusammensetzung/Kapsel** |
|---|---|
| Methylendiol-Steroid | 0,2 mg |
| Ascorbinsäure | 2,5 mg |
| Ethanol | 10,0 mg |
| Dehydroepiandrosteron (DHEA) | 50,0 mg |
| Mittelkettige Triglyceride (Miglyol 812) | 37,3 mg |
| Gesamtmasse 100.0 mg | |

Die Kapselhülle wird in üblicher Weise unter Verwendung von Gelatine hergestellt.

Es wurden in üblicher Weise Weichgelatinekapseln mit vorstehenden Zusammensetzungen hergestellt.

### Beispiel 2: Herstellung eines erfindungsgemäßen Arzneimittels aus DHEA und 14α,15α-Methylenestra-1,3,5(10),8-tetraen-3,17α-diol als transdermales therapeutisches System.

| | |
|---|---|
| 1,50 g | 14α,15α-Methylenestra-1,3,5(10),8-tetraen-3,17a-diol |
| 2,70 g | DHEA |

werden in 20,0 g Aceton gelöst, zu 299,4 g MA-24A (32%-ige Lösung in Heptan) gegeben, und zu einer klaren Masse verrührt. Die Masse wird mit einem Beschichtungsstab auf einem Silicium-beschichteten Polyesterfilm verteilt. Dann wird die Masse getrocknet, wodurch ein Haftfilm erhalten wird, der eine aktive Substanz enthält. Der Film wird mit einem Polyethylenfilm bedeckt. Dann werden einzelne Stücke mit einer Fläche von 10 bis 25 cm² (vorzugsweise 15 cm²) ausgestanzt.

## Patentansprüche

1. Arzneimittel zur Behandlung der klimakterischen Beschwerden infolge gleichzeitigen Estrogen und Androgenmangels in der Postmenopause, das nicht zur Proliferatien der Gebärmutterschleimhaut führt, umfassend
a) Steroid der Formel (1) 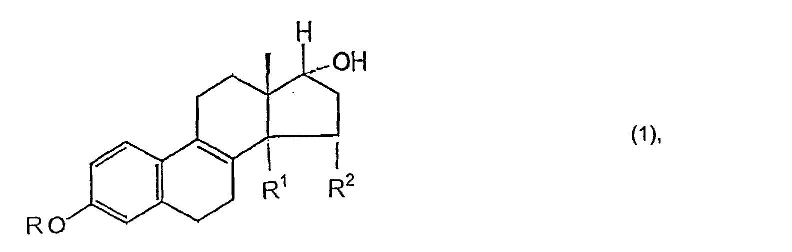 in der
R für Wasserstoff oder ein C₁-C₄-Alkyl und
R¹ und R² für Wasserstoff stehen oder R¹ und R² identisch sind und CH₂ bedeuten, sowie
b) Dehydroepiandrosteron und/oder einen Dehydroepiandrosteron-Ester und/oder andere Derivate des Dehydroepiandrosterons.

2. Arzneimittel nach Anspruch 1, wobei das Steroid die Formel (2) 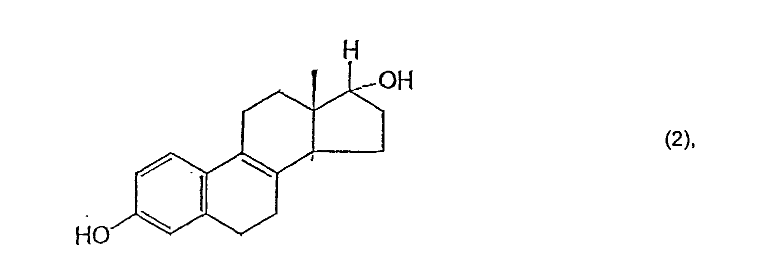 aufweist.

3. Arzneimittel nach Anspruch 1, wobei das Steroid die Formel (3) 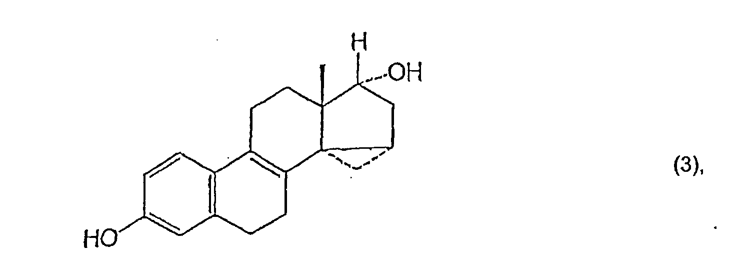 aufweist.

4. Arzneimittel nach einem der vorhergehenden Ansprüche, wobei es als oral oder transdermal verabreichbare Formulierung vorliegt.

5. Arzneimittel nach einem der vorhergehenden Ansprüche, wobei es als Kombinationspräparat mit zwei Applikationsformen vorliegt, wobei eine Applikationsform das Steroid und die andere Applikationsform das Dehydroepiandrosteron und/oder den Dehydroepiandrosteron-Ester enthält und/oder das Dehydroepiandrosteron-Derivat.

6. Arzneimittel nach einem der vorhergehenden Ansprüche, wobei das Steroid mit Ascorbinsäure stabilisiert ist.
